# EUROPEAN PATENT APPLICATION

(11) **EP 4 248 913 A1**
(43) Date of publication of application: **27.09.2023**
(21) Application number: 22164233.3
(22) Date of filing: 24.03.2022
(51) Int. Cl.: A61F 2/24, A61F 2/06

(54) **DEVICE FOR HEART VALVE REPAIR**

(71) Applicant: Université catholique de Louvain, 1348 Louvain-la-Neuve (BE)
(72) Inventor: BOLLEN, Xavier, 1200 Woluwe-Saint-Lambert (BE); de KERCHOVE de DENTERGHEM, Laurent, 1200 Woluwe-Saint-Lambert (BE)
(74) Representative: Icosa

(57) **Abstract**

The present invention relates to a device for heart valve repair configured to be implanted around a heart valve of a subject, the heart valve comprising an annulus and at least two leaflets, the device comprising: a base configured to fit around the annulus of the heart valve, so as to limit a radially outward displacement of said annulus; at least two elongated members extending from the base, each elongated member extending parallel to a longitudinal axis and ending in a free end opposite to the base and being sized to extend between the annulus of the heart valve and a commissure between the at least two leaflets; a securing member configured to join at least two free ends, so as to prevent an outward displacement of said free ends when the device is implanted around the heart valve.

## Description

### FIELD OF INVENTION

The present invention relates to the field of heart valve repair and, more precisely, to heart valve repair devices configured to be implanted around a heart valve of a subject comprising an annulus and at least two leaflets.

The invention also relates to a corresponding manufacturing method.

### BACKGROUND OF INVENTION

The aortic valve is one of the four heart valves in the human body, and it ensures the unidirectional blood flow from the left ventricle to the aorta.

The aortic valve comprises three leaflets attached to the aortic root, which extends between the aortic annulus and the sinotubular junction and comprises the sinuses of Valsalva.

Abnormalities in one or more of these structures may lead to aortic regurgitation. Aortic valve regurgitation is a condition that occurs when the valve does not close properly, thereby causing a retrograde blood flow (or leakage) from the aorta backward into the left ventricle. Moreover, congenital abnormality such as a bicuspid aortic valve may also contribute to aortic valve regurgitation.

The main treatments for aortic valve diseases associated with regurgitation are valve repair and valve replacement.

Mechanical prostheses require the use of anticoagulants. Anticoagulants are associated with thromboembolic and hemorrhagic risks, which may explain the reduction in the life expectancy observed in patients receiving mechanical valve prostheses.

On the other hand, biological prostheses prevent anticoagulation but they can degenerate over time and this phenomenon can be extremely fast in young patients.

In addition to the aforementioned risks, mechanical and biological valves may further cause conduction disturbances, permanent pacemaker implantation, and patient-prosthesis mismatch.

In most cases, valve sparing or valve repair are preferable to valve replacement. However, valve repair procedures are currently implemented only in a limited number of centers, mainly because the procedure is technically complex and difficult to standardize.

Moreover, currently available devices for valve repair typically restore the functionality of one specific structure of the aortic valve (*e.g.*, reducing annulus dilation) but fail to take into account the requirements and specificities of the neighbor structures.

The goal of the present invention is to provide a device for aortic valve repair which provides optimal mechanical support to the aortic valve and its neighbor structures.

It is also a goal of the present invention to provide a device for aortic valve repair which is easy to implant with standardized procedures.

Furthermore, the present invention aims at providing devices and methods for heart valve re-implantation and which may be associated with a remodeling technique.

### SUMMARY

To this end, the invention thus relates to a device for heart valve repair configured to be implanted around a heart valve of a subject, the heart valve comprising an annulus and at least two leaflets, the device comprising:
- a base configured to fit around the annulus of the heart valve, so as to limit a radially outward displacement of said annulus;
- at least two elongated members extending from the base, each elongated member extending parallel to a longitudinal axis and ending in a free end opposite to the base and being sized to extend between the annulus of the heart valve and a commissure between the at least two leaflets;
- a securing member configured to join at least two free ends, so as to prevent an outward displacement of said free ends when the device is implanted around the heart valve.

In one embodiment, the securing member is a flexible band.

In one embodiment, each elongated member is surrounded by a fabric, preferably at least one strip of fabric.

In one embodiment, the device does not have an axis of symmetry.

In one embodiment, the base is annular and the elongated members are spaced apart such that the angles defined by the rays emanating from the center of the annular base to two consecutive elongated members and are comprised between 110° and 190°.

In one embodiment, the device is axisymmetric with respect to the longitudinal axis.

In one embodiment, the longitudinal axis is perpendicular to the base.

In one embodiment, the total length of the device measured along the longitudinal axis is comprised between 20% and 40% of the total length of the base.

In one embodiment, the device comprises at least two adjacent arc-shaped members each comprising a curved portion and two linear portions terminating in two respective free ends, wherein at least one linear portion of each arc-shaped member is joined to the linear portion of the adjacent arc-shaped member at the free end, such that:
- the base of the device is formed by the curved portions of the arc-shaped members, and
- the elongated members of the device are formed by the linear portions of the arc-shaped members.

In one embodiment, the two linear portions of adjacent arc-shaped members are joined together along a length of 75% or less of the total length of the linear portion.

In one embodiment, the device is configured to be wrapped from an open configuration in which the device is roughly planar and the base has an elongated shape, to a wrapped configuration in which the device is cylindrical and the base has an annular shape.

In one embodiment, each pair of adjacent free ends except one is made by two free ends joined with continuity of material, and one pair of adjacent free ends is made by two free ends joined with a detachable link, such that the device presents:
- an open configuration in which the device is roughly planar, and
- a wrapped configuration in which the two adjacent free ends joined with the detachable link are linked so as to form a cylindrical device.

In one embodiment, the elongated members and the base are made of metal or metal alloy or polymers, preferably obtained from molding or additive manufacturing from a powder or from wires.

In one embodiment, the elongated members are covered with a polyester fabric.

The present invention further relates to an aortic root replacement prosthesis comprising a tubular portion having a first end configured to be implanted as replacement of a portion of an ascending aorta which begins at the aortic root and a second end configured to be implanted as replacement of the aortic root, said second end forming at least two lobes, each lobe closely fitting the space between two adjacent elongated members of the device according to any one of the embodiments described hereinabove.

The present invention further relates to a method for repairing an aortic root structure, the method comprising:
- providing the device disclosed hereabove and configured to be wrapped from an open configuration to a wrapped configuration; and wrapping externally around an aortic root while passing one of the two free ends of the pair of adjacent free ends joined with a detachable link under the left coronary and the other free ends of said pair under the right coronary artery;
- joining said two free ends via the detachable link, such that the device is in a wrapped configuration in which the base surrounds the aortic annulus and each elongated member fits an interleaflet space and extends between the annulus and the commissure between the at least two leaflets;
- securing the base of the device to the aortic root with suturing stitches; and
- securing each free end of the device to the aortic root with suturing stitches.

The present invention further relates to a method for repairing a tricuspid or bicuspid aortic valve, the method comprising:
- dissecting the aortic root;
- inserting the aortic root replacement prosthesis described above such that it surrounds the aortic annulus and the commissures between the valve leaflets are inside the second end of the tubular portion, each commissure facing a corresponding elongated member;
- securing each free end to a respective tip of the commissures with suturing stitches.

### DEFINITIONS

In the present disclosure, the following terms or expressions have the following meanings:
- "***about***" is used herein to mean approximately, roughly, around, or in the region of. When the term "*about*" is used in conjunction with a numerical range, it modifies that range by extending the boundaries above and below the numerical values set forth. When the term "*about*" is used preceding a figure means plus or less 10% of the value of said figure.
- "***aortic root apparatus***" refers to the aortic annulus, the aortic leaflets, the sinuses of Valsalva, and the sinotubular junction;
- "***total length of the device***" refers to the length of the device measured along the longitudinal axis of the elongated members of the device, said total length including the length of the elongated members and the height of the base of the device, i.e., the total length being Lm + h;
- "***total length of the base***" refers to the length of the base of the device measured in an unfolded state of the base, *i.e.,* the perimeter of the base (*e.g.*, for a circular base, the total length of the base is the circumference);
- "***valve sparing***" refers to a repair surgical procedure in which the aortic root is reconstructed while preserving the tissues of the native aortic valve, examples of said valve sparing being reimplantation and remodeling;
- "***valve reimplantation***" refers to a valve-sparing surgical procedure of the aortic valve in which the dilation of the aortic annulus and that of the sinotubular junction is constrained, the sinuses of Valsalva are created and the aortic valve is reimplanted (*i.e.*, resuspended) inside a tube graft;
- "***valve remodeling***" refers to a valve-sparing surgical procedure of the aortic root in which the sinotubular junction is constrained (*e.g.*, wherein the aortic root is replaced by a tube and the native aortic valve is preserved);
- "***valve repair***" refers to surgical procedure in which the leaflets of the aortic valve are repaired; and wherein the native tissues of the aortic root are kept or removed, and which may optionally be combined with a valve sparing surgical procedure.
- "***valve replacement***", refers to the removal of the diseased valve, and its replacement with a mechanical or biological prosthesis.

### BRIEF DESCRIPTION OF THE DRAWINGS

**Figure 1** is a perspective view of an embodiment of a device 1 for heart valve repair according to the invention.
**Figure 2A** is a perspective view of a sub-assembly of the device 1 according to an embodiment.
**Figure 2B** is a perspective view of a sub-assembly of the device 1 according to an embodiment.
**Figure 3** is a top view of the sub-assembly of **figure 2B**.
**Figure 4A** is a lateral view of a sub-assembly of the device 1 according to an embodiment in which the device 1 is not axisymmetric.
**Figure 4B** is a frontal view of the sub-assembly of **figure 4A**.
**Figure 5A** is a lateral view of a sub-assembly of the device 1 according to an embodiment.
**Figure 5B** is a frontal view of the sub-assembly of **figure 5A**.
**Figure 6A-6C** are perspective views of a sub-assembly of the device 1 according to an embodiment in which the device 1 comprises three arc-shaped members joined together along different lengths L.
**Figure 7** is a lateral view of the device 1 according to an embodiment.
**Figure 8A** is a perspective view of an aortic root replacement prosthesis 2 according to an embodiment in which the aortic root replacement prosthesis 2 comprises three lobes.
**Figure 8B** is a is a lateral view of the aortic root replacement prosthesis 2 of **figure 8A**.
**Figure 8C** is a is a frontal view of the aortic root replacement prosthesis 2 of **figure 8A**.
**Figure 9A** is a perspective view of an aortic root replacement prosthesis 2 according to an embodiment in which the aortic root replacement prosthesis 2 comprises two lobes.
**Figure 9B** is a is a lateral view of the aortic root replacement prosthesis 2 of **figure 9A**.
**Figure 9C** is a is a frontal view of the aortic root replacement prosthesis 2 of **figure 9A**.
**Figure 10A** is a perspective view of a sizer 3 according to an embodiment.
**Figure 10B** is a is a frontal view of the sizer 3 of **figure 10A**.
**Figure 11A** is a perspective view of the sizer 3 according to another embodiment.
**Figure 11B** is a is a frontal view of the sizer 3 of **figure 11A**.

### DETAILED DESCRIPTION

### Device

The present invention relates to a device 1 for heart valve repair, in particular aortic valve repair, configured to be implanted around a heart valve of a subject, the heart valve comprising an annulus and at least two leaflets.

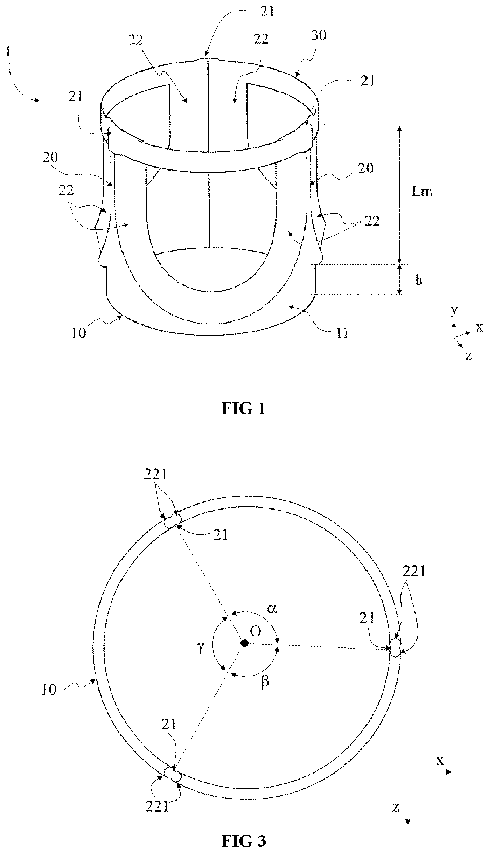

**Figure 1** shows a device 1 according to the invention.

As shown in **figure 1**, the device 1 comprises a base 10, at least two elongated members 20 extending from said base 10, and a securing member 30.

Once implanted, the base 10 fits around the annulus of the heart valve, so as to limit a radially outward displacement of the same (*i.e.*, an unwanted increase of the annulus diameter). Thus, the base 10 is a structure with a section corresponding to the shape of the annulus and a dimension perpendicular to the section corresponding to the area where the device 1 is anchored. The base 10 of the device 1 has a height h corresponding to the height of the valve annulus which is supported by said base 10 (*e.g.*, for an aortic valve, corresponding to the area between the ventriculo-aortic junction and the basal attachments (*i.e.*, the valve attachment), or nadirs to which the device 1 is anchored).

The elongated members 20 are sized to extend between the annulus of the heart valve and a commissure between the valve leaflets.

The securing member 30 is configured to join together the elongated members 20, so as to limit their displacement. In particular, the securing member 30 avoids an outward displacement of the free ends 21 of the elongated members 20 (*i.e.*, the extremities of the elongated members 20 opposite to the base 10) when the device 1 is implanted around the heart valve (*i.e.*, a displacement outside of the sinotubular junction). Therefore, the securing member 30 allows to remodel or constrict the sinotubular junction to a specific size, corresponding to the diameter of the device 1 (which will be described later).

For instance, the securing member 30 may be made of a flexible band.

In this case, the securing member 30 has a width comprised between 2 mm and 6 mm. Preferably, said width is equal to about 4 mm.

The securing member 30 is preferably made of a polymeric material. For instance, it may be made of a polymeric fabric such as polyethylene terephthalate (e.g., Dacron^{™}) or expanded polytetrafluoroethylene (*e.g.*, GoreTex^{™} or Teflon^{™}). These materials have the advantage of being soft and flexible.

Moreover, the device 1 may comprise one or more strips 11, 22. For instance, the device 1 may comprise lateral strips 22 surrounding the elongated members 20.

Advantageously, the lateral strips 22 allow to compress a larger surface of the aortic root (more precisely, the whole interleaflet triangle) of an aortic valve when the device 1 is implanted in the subject. Moreover, these strips 22 limit the displacement of the elongated members 20 and make the device 1 more resistant to wear.

The lateral strips 22 may be flexible bands, for instance made of tissue or fabric. In this case, the lateral strips 22 preferably have a width comprised between 2 mm and 6 mm, or equal to about 4 mm. The width of the lateral strips 22 and that of the securing member 30 may be identical.

Preferably, the elongated members 20 are embedded inside (*i.e.*, completely surrounded by) the lateral strips 22 **(****figure 1**).

In addition, the device 1 may comprise a basal strip 11.

The lateral strip 22, the basal strip 11, and the securing member 30 are preferably made of the same material.

Said basal strip 11 may have a width which is equal to the width of the lateral strips 22.

Advantageously, this basal strip 11 allow to exert a higher compression on the valve annulus. Moreover, the basal strip 11 allows to provide an increased surface for the attachment of the device 1 to the valve annulus. For instance, it may house anchoring stitches arranged on the basal strip 11 such that they match complementary anchoring stitches on the annulus.

In the example of **figure 1**, the basal strip 11 completely surrounds the base 10 of the device 1.

As aforementioned, the base 10 is configured to fit around the annulus of the heart valve. To this end, the base 10 is hollow, for instance, annular.

Accordingly, the base 10 has an internal diameter and an external diameter. The internal diameter of the base 10 is preferably comprised between 24 mm and 34 mm.

The device 1 may be provided in various sizes having different internal diameters (*e.g.*, with a difference of 2 mm between the internal diameter of one device size and the internal diameter of the consecutive device size), to give the possibility, to the surgeon, to select the best internal diameter based on the patient anatomy.

Moreover, the possibility to select the internal diameter of the device 1 in this range advantageously allow to predict and control the diameter of the valve annulus after implantation of the device 1. For instance, the diameter of the aortic annulus may be calculated based on the internal diameter of the base 10 and the thickness of the tissues constituting the aortic root and aortic annulus (typically about 6 mm to 7 mm).

The elongated members 20 extend from the base 10 and each of them ends in a free end 21 opposite to the base 20.

Preferably, the elongated members 20 are parallel, *i.e.*, they extend from the base 10 parallel to the same longitudinal axis y. In this case, the longitudinal axis y is perpendicular to the base 10.

Advantageously, the diameter of a device 1 comprising parallel elongated members 20 is constant (*i.e.*, the device 1 has a cylindrical profile). Therefore, the pressure exerted on the tissues of the subject, when the device 1 is implanted, is also uniform. Accordingly, this embodiment avoids the risk of excessively solicitating the sinotubular junction or perforating the aortic wall (for instance, with the free ends 21 of the elongated members 20).

The device 1 preferably has a total length (*i.e.*, Lm + h) which is comprised between 20% and 40% of the total length of the base 10. As will be later described, the base 10 may have different shapes (*e.g.*, elongated or annular). Accordingly, by "*total length of the base*" it is meant the length of the base 10 when unfolded (*e.g.*, the circumference, if the base is circular).

As aforementioned, the device 1 is configured to be implanted around a heart valve. Thus, the base 10 of the device 1 corresponds to a section of said heart valve (*i.e.*, it corresponds to a section of the vessel containing said heart valve, *e.g.*, the aorta), and the total length of the device is measured along a longitudinal axis y which corresponds to the axis of the heart valve (*i.e.*, it corresponds to the axis of the vessel containing said heart valve, *e.g.* the axis of the aorta). Moreover, the base 10 is placed around the heart valve such that the height h of the base 10 is along said longitudinal axis, *e.g.*, the axis of the aorta.

Accordingly, the total length of the device 1 (measured along the longitudinal axis y) is given by the sum of the length Lm of the elongated members 20 and the height h of the base 10 (Lm + h).

The length Lm of each elongated member 20 is measured between its free end 21 and the point of insertion of said elongated member 20 in the base 10.

For an annular or circular base (**figure 1**), said total length of the device 1 is comprised between 80% and 120% of the internal diameter of the base 10. Preferably, said total length is equal to about 90% of the internal diameter of the base 10.

These lengths are suitable for obtaining elongated members 20 which extend between the annulus of the heart valve and the commissure between the valve leaflets, thereby providing mechanical support to the aortic valve as well as its neighbor structures.

The elongated members 20 may have a cross section of any shape, such as circular, squared, elliptic, semicircular or rectangular.

Preferably, the elongated members 20 have a circular cross-section.

The diameter of the cross-section of the elongated members 20 is comprised between 0.7 mm and 1.2 mm. Preferably, said diameter is equal to about 0.9 mm.

Moreover, the elongated members 20 preferably have the same length Lm.

The device 1 may be axisymmetric, *i.e.*, the longitudinal axis y previously described is also an axis of symmetry of the device 1.

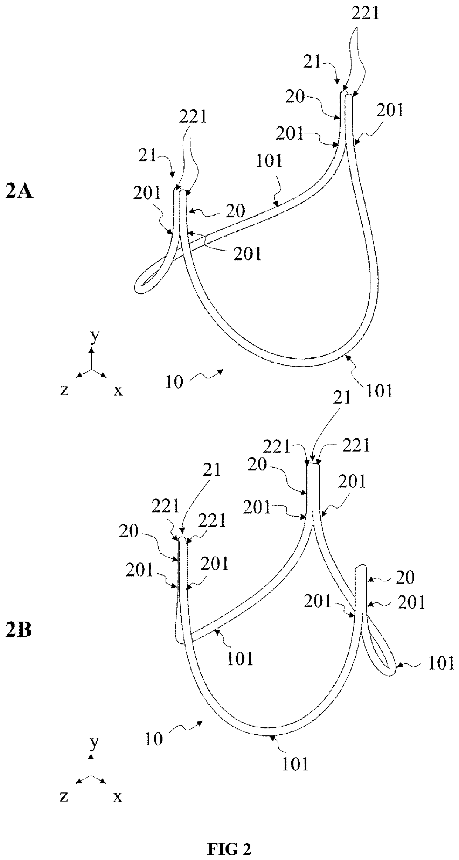

**Figure 2** illustrates two exemplary axisymmetric devices 1. For intelligibility purposes, only the skeleton (*i.e.*, the base 10 and the elongated members 20) of the device 1 is therein illustrated.

Preferably, the device 1 has a two-fold (**figure 2A**) or three-fold (**figure 2B**) degree of symmetry.

The skeleton of the device 1 may be made of biomaterials such as nickel titanium alloy (Nitinol^{™}), polymers such as polyether ether ketone (PEEK), or stainless steel. Preferably, the skeleton of the device 1 is made of nickel titanium alloy, as this material allows to provide elevated flexibility and resistance.

In this case, the base 10 of the device 1 is also axisymmetric, and the elongated members 20 are separated by the same angular distance.

More precisely, each pair of consecutive elongated members 20 forms an angle α, β, γ with a center O of the base 10 and, in an axisymmetric device, these angles α, β, γ are equal. For instance, in a device 1 with two elongated members 20, said elongated members 20 may form two angles α, β equal to 180° (**figure 2A**), or, in a device 1 with three elongated members 20, said elongated members 20 may form three angles α, β, γ equal to 120° **(****figure 2B**). The former is particularly adapted for repairing a symmetric bicuspid valve tricuspid valve, and the latter for repairing a tricuspid valve.

Said angles α, β, γ are better shown in **figure 3**, which is a top view of the device of **figure 2B**. In this example, the elongated members 20 form three angles α, β, γ equal to 120°.

Coming back to **figure 2A**, the device 1 therein illustrated has a base 10 formed by two identical curved portions 101. In particular, the skeleton of this device 1 is made of two arc-shaped members, each arc-shaped member comprising one curved portion 101 and two linear portions 201 terminating in two respective free ends 221.

Adjacent linear portions 201 are joined together at the free end 221, such that:
- the base 10 of the device 1 is formed by the two curved portions 101 of the arc-shaped members, and
- the elongated members 20 of the device 1 are formed by the linear portions 201 of the arc-shaped members.

Moreover, the free end 21 of each elongated member 20 is formed by two joined free end 221.

The device of **figure 2B** is also an axisymmetric device 1 comprising identical arc-shaped members. In this case, the device 1 comprises three arc-shaped members, so that the base 10 is formed by three curved portions 101, and three elongated members 20 are formed by six linear portions 201.

In some examples, the device 1 is not symmetric. For instance, the elongated members 20 may form different angles α with the center O of the base 10 (not shown).

Preferably, the angle α is comprised between 110° and 190°.

In a device comprising two elongated members 20, the angle α is preferably comprised between 170° and 190°. In this case, angles α different than 180° (*e.g.*, 175° and 185°) may be provided in a device 1 for repairing an asymmetric bicuspid valve.

In addition, the non-symmetry of the device 1 may be due to an asymmetrical shape of the base 10.

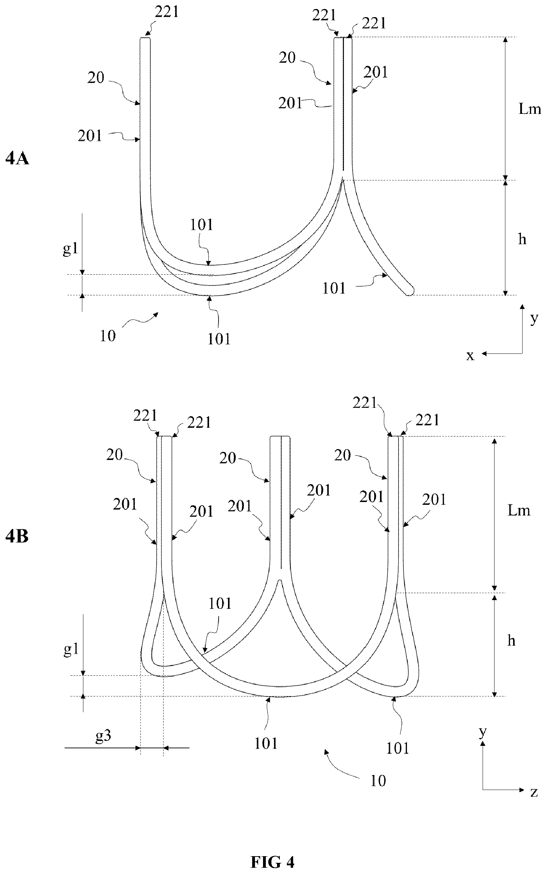

**Figures 4A** and **4B** are views of a non-axisymmetric device 1 in which the base 10 is not symmetric.

More precisely, the base 10 of this device comprises curved portions 101 which have different heights h. In other words, a vertical gap g1 exists between the shortest curved portion 101 and the longest curved portion 101.

By providing a vertical gap g1 in the base 10, it is possible to provide a device 1 which better fits an asymmetric aortic root.

As can be seen in **figure 4B**, the device 1 may also present a bend. In this case, the curved portions 101 of the base 10 slightly protrude from the cylinder including the linear portions 201, thereby forming a small gap g3. This particular shape may be obtained for instance by manufacturing the arc-shaped members by wrapping three tubes around a cylinder.

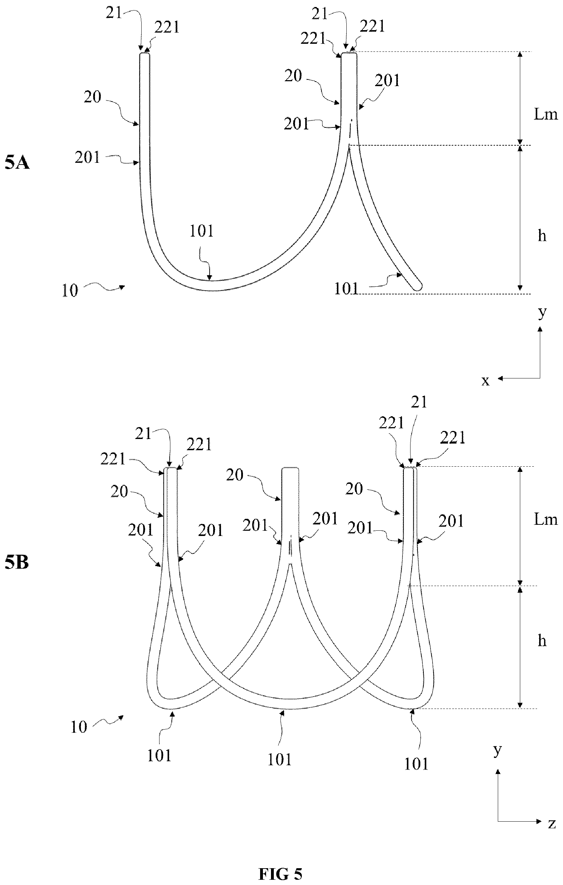

**Figures 5A** and **5B** are views of a symmetric device 1 in which the base 10 does not present a vertical gap g1.

Overall, a device 1 made of arc-shaped members (with or without vertical gap g1) better reproduces the anatomy of the aortic valve. Indeed, the "U" shape of each member perfectly matches, once implanted, the curvature of a respective leaflet attachment profile.

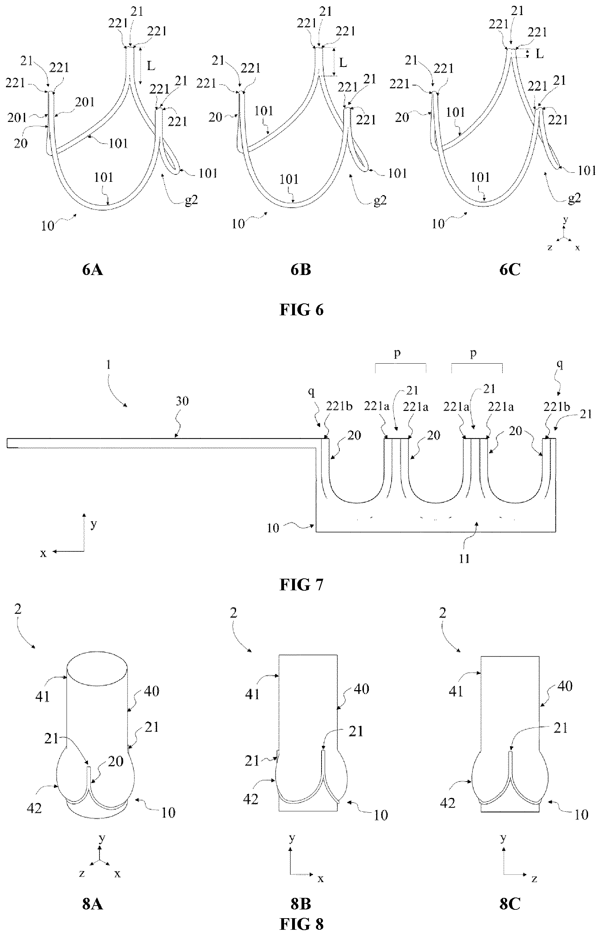

**Figure 6** illustrates three examples of devices made of arc-shaped members. More precisely, **figures 6A**, **6B** and **6C** illustrates three different axisymmetric devices 1 comprising elongated members 20 which form an angle of 120° (not shown) with the center O of the base 10, and wherein the base 10 does not present a vertical gap g1.

In these examples, adjacent arc-shaped members are joined together along a fraction of the linear portions 201 and, more precisely, along a length L which is smaller than 75% of the total length Lm of the linear portion 201.

As can be seen in **figure 6**, to decreasing joining lengths L (*i.e.*, going from **figure 6A** towards **figure 6C**) correspond increasing horizontal gaps g2 (*i.e.*, measured on a plane parallel to xz) between joined linear portions 201. In other words, with smaller joining lengths L, the three "U" which form the skeleton of the device 1 are more spaced apart.

**Figure 7** is a frontal view of a device 1 according to the invention which presents two configurations.

More precisely, this device 1 is configured to be enfolded from an open configuration **(****figure 7**) in which the device 1 is roughly planar (*i.e.*, the configuration of the device when it lies on a flat surface) to a wrapped (or closed) configuration in which the device 1 is cylindrical (**figure 1**).

By "*roughly planar*" it is meant having two dimensions (*e.g.*, a length and a width) that are greater than a third dimension (*e.g.*, a height). A *roughly planar* object may be flat, or may have slight curvatures or bent.

Accordingly, the expression "*roughly planar*" may refer to a device 1 in which the arc-shaped portions are flat (*i.e.*, without a bend between the linear positions 201 and the curved portion 101), or a device 1 in which the arc-shaped portions present a bend **(****figure 4B**).

Coming back to **figure 7**, the advantage of a device 1 having an open and a wrapped configuration, is the possibility to treat isolated aortic insufficiency (AI) where the aortic root is not replaced.

In particular, the device 1 in its open configuration is passed below the coronary arteries that raise from the aortic root. The open configuration allows to pass the elongated members 20 below the coronaries and wrap the device 1 around the aortic root into its wrapped configuration.

More precisely, in the device 1 of **figure 7** each pair p of adjacent free ends 221 except one is made by two free ends 221a joined with continuity of material, and one pair q of adjacent free ends 221 is made by two free ends 221b joined with a detachable link.

Moreover, in the open configuration the securing member 30 has a free extremity and another extremity attached to a free end 221.

To put the device in its wrapped configuration, the two adjacent free ends 221b are attached by means of the detachable link.

As a result, in its open configuration the device 1 is roughly planar, and the base 10 has an elongated shape, and in its wrapped configuration it is cylindrical, and the base 10 has an annular shape.

A device 1 having such open and wrapped configurations avoids to cut the coronary arteries. Accordingly, it permits to decrease the length of the surgical procedure since an anastomosis of the coronaries is avoided.

In some examples, the device 1 may only present a wrapped configuration.

Advantageously, a device 1 presenting only a wrapped configuration may be employed in surgical procedures where the coronaries are detached from the aortic root because sinuses of Valsalva are resected (*e.g.*, aneurism).

### Aortic root replacement prosthesis

The present invention further relates to an aortic root replacement prosthesis 2 (*e.g.*, a Valsalva prosthesis) comprising a device 1 above described.

**Figure 8** illustrates an aortic root replacement prosthesis 2 of the invention according to a first embodiment. **Figure 8A** is a perspective view of the prosthesis 2, and **figures 8B** and **8C** are a frontal and a lateral view of the prosthesis 2, respectively.

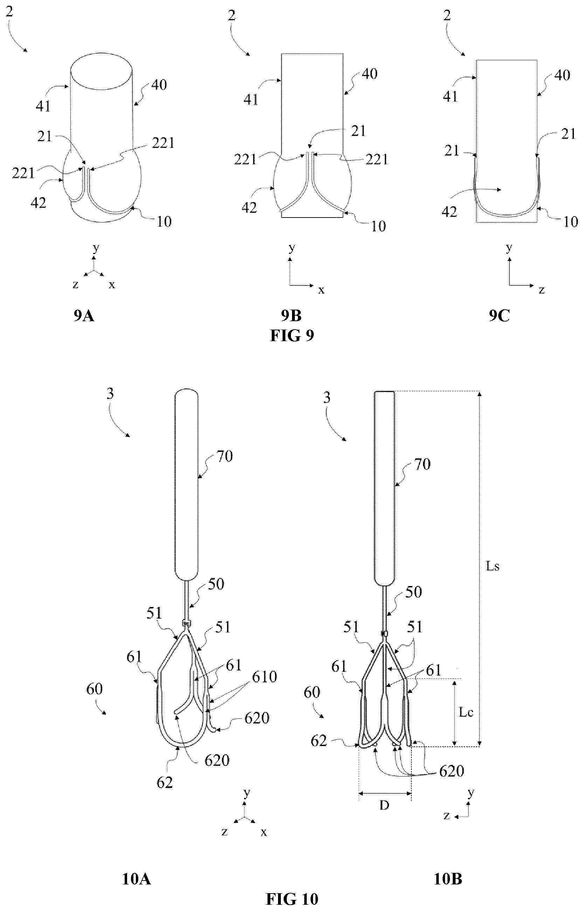

**Figure 9** illustrates an aortic root replacement prosthesis 2 according to a second embodiment. **Figure 9A** is a perspective view of the prosthesis 2, and **figures 9B** and **8C** are a frontal and a lateral view of the prosthesis 2, respectively.

The aortic root replacement prosthesis 2 comprises a tubular portion 40 (e.g., a Dacron^{™} graft) having a first end 41 configured to replace a portion of the ascending aorta which begins above the aortic root and a second end 42 forming at least two lobes configured to replace the aortic root.

The prothesis 2 of **figure 8** comprises three lobes.

The prothesis 2 of **figure 9** comprises two lobes.

Each of said lobes closely fits the space between two adjacent elongated members 20.

In the aortic root replacement prosthesis 2, the first end 41 of the tubular portion 40 has the role and function of securing member 30.

The design of this aortic root replacement prosthesis 2 is particularly adapted for surgical procedures in which the aortic root is resected and the coronary arteries are detached from the aortic root. In this case, the detached coronary arteries may be reattached (*i.e.*, sutured) on the lobes provided on the second end 42 of the aortic root replacement prosthesis 2.

The aortic root replacement prosthesis 2 may comprise a device 1 comprising two or three free arc-shaped members as previously described. In this case, each pair of adjacent free ends 221 is joined with continuity of material.

For example, two adjacent free ends 221 may be directly joined to each other (*e.g.*, attached together). Alternatively, two adjacent free ends 221 may be joined to each other via the tubular portion 40, as in the example of **figure 9**. In particular, in the example of **figure 9** the aortic root replacement prosthesis 2 comprises two lobes. However, a configuration with the adjacent free ends 221 joined to each other via the tubular portion 40 may also be provided on an aortic root replacement prosthesis 2 with three lobes previously described.

By joining two adjacent free ends 221 via the tubular portion 40, a space is created between said free ends 221 which allows to widen the commissure when the aortic root replacement prosthesis 2 is implanted.

### Material

The skeleton of the device 1 may be made of metal or metal alloy or polymers. Preferably, it is obtained from molding or from additive manufacturing, from a powder (*e.g.*, selective laser sintered powder) or from wires.

For example, the skeleton of the device 1 is obtained from powder injection molding of a metal or metal-alloy or polymer powder (preferably a stainless-steel powder) or from thermoforming a tube (such as a nickel titanium wire) into a mold.

In other examples, the skeleton may be obtained from a metal or metal-alloy tube (preferably a nickel titanium wire) by laser-cutting.

These materials and manufacturing techniques are suitable to provide the skeleton of the device 1 of **figures 2**, **3**, **4**, **5** and **6**, which comprises arc-shaped members.

The skeleton of the device 1 made of metal or metal alloy or polymers may be covered with a fabric, preferably a polyester fabric. This allows to obtain elongated members 20 which are completely embed inside the strips 11, 22 previously described.

As aforementioned, the device 1 further comprise a securing member 30. Said securing member may be attached to the skeleton of the device 1 such that it joins together at least two free ends 21.

For example, the securing member 30 may be attached the to the free ends 21 of the elongated members 20 by crimping or tight suturing (*e.g.*, suturing with Dacron^{™} patches).

These attaching techniques are suitable to attach a securing member 30 consisting in a flexible band (**figures 1** and **7**).

### Sizer

The present invention further relates to a sizer 3 for calibrating the device 1, *i.e.*, selecting the size of device 1 (in particular, the diameter of the base 10) prior implantation.

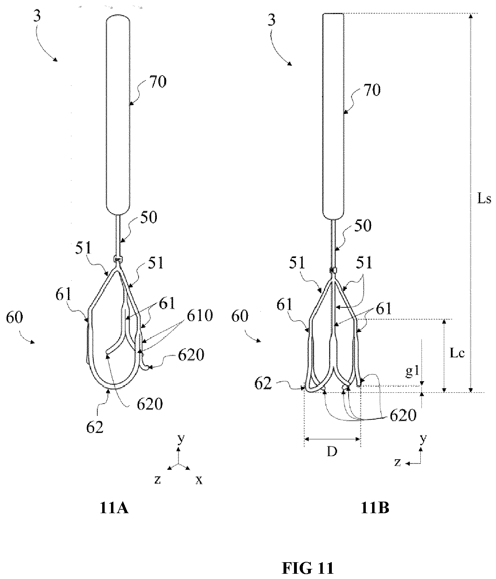

Two examples of sizer 3 are shown in **figures 10** and **11**.

One option in the domain of aortic root remodeling is the implantation of an external annuloplasty ring. These rings do not provide an optimal mechanical support to the aortic valve and its neighbor structures.

Moreover, the calibration of conventional annuloplasty rings consists in measuring the native aortic annulus with a cylindrical dilator. A drawback of this calibration is that it is based on the dimension of the annulus. Nevertheless, the annulus is dilated (pathological) in the setting of aortic valve insufficiency or root aneurism. Therefore, using the dimension of the annulus as a reference for selecting the size of the annuloplasty ring is misleading.

Advantageously, the present device 1 allows to overcome these problems as it may be calibrated with the sizer 3. The sizer 3 of the invention allows to use, as a reference for the valve size of the device 1, the dimension of the valve.

With reference to **figure 10**, the sizer 3 of the invention comprises a main body formed by a rod 50 extending along a longitudinal direction y and ending in a calibrating portion 60.

The length Ls of the sizer is preferably comprised between 180 mm and 250 mm.

The calibrating portion 60 occupies 1/2 to 1/4 of the length Ls of the sizer. Preferably, the calibrating portion 60 has a length Lc comprised between 35 mm and 50 mm. The inner diameter D of the calibrating portion 60 is comprised between 22 mm and 38 mm. The outer diameter of the calibrating portion (not illustrated) is defined by the inner diameter D and the thickness of the calibrating portion, the latter being typically comprised between 1 mm and 3 mm.

In what follows, the term "*proximal*" refers to an element which is closer to the calibrating portion 60 of the sizer 3, the term "*distal*" refers to an element which is closer to an extremity of the sizer 3 opposite to the calibrating portion 60.

The sizer 3 may also comprise a handle 70 on its distal end which allows manual insertion and removal of the sizer 3. In **figure 10**, the handle 70 has the form of a sleeve surrounding the rod 50.

The calibrating portion 60 of the sizer 3 allows to determine the diameter of the aortic valve of the subject prior implantation of the device 1, so as to select the optimal size of the device 1.

Accordingly, the shape of the calibrating portion 60 may vary depending on whether the device 1 to be implanted is axisymmetric or not.

For instance, the sizer 3 of **figure 10** is particularly adapted for determining the size of an axisymmetric device 1 (*e.g.*, the device of **figure 1**), whereas the sizer 3 of **figure 11** is particularly adapted for determining the size of a device 1 having a base 10 which comprises curved portions 101 with different heights h (*e.g.*, the device of **figure 4**).

The rod 50 branches out into at least two branches 51 which connect the rod 50 with the calibrating portion 60. More precisely, each branch 51 connects the rod 50 to a respective elongated member 61 provided on the calibrating portion 60.

The calibrating portion 60 has a three-dimensional scalloped shape comprising at least two curved elements 62 which are joined, through the elongated member 61 and the branches 51, to the rod 50 of the sizer 3.

The curved element 62 and the elongated members 61 of the calibrating portion 60 may be similar to the elongated member 21 and the curved portions 101 of the device 1. In this case, each elongated member may comprise two linear portions 610, each of which is joined to a linear portion 610 of an adjacent elongated member 61 through a curved element 62.

As can be seen in **figures 10** and **11**, each elongated member 61 forms with the two curved elements 62 to which it is joined, a cusp. These cusps are configured to be abutted against the commissure of the heart valve and the elongated member 61 are configured to follow the leaflets' insertion.

Moreover, as shown in **figures 10** and **11**, one or more curved elements 62 may be discontinuous, *i.e.*, they comprise an opening, delimited by two tips 620. In this case, the two elongated elements 61 proximate to said opening are not joined together.

Preferably, for a sizer comprising three cusps, said opening is provided on at least two cusps.

The opening advantageously permits, during introduction of the sizer 3 and while said sizer 3 is in position in the valve, the passage of the right and left coronaries that are attached to the aortic root therethrough.

### Methods of implantation

As aforementioned, the device 1 is configured to be implanted around a heart valve of a subject.

As aforementioned, the device 1 may have only a wrapped configuration (for instance when it forms, with the tubular portion 40, the aortic root replacement prosthesis 2) or it may present both an open and a wrapped configuration.

The former may be implanted via a valve sparing reimplantation procedure, and the latter via an isolated repair of a tricuspid or bicuspid aortic valve.

The method for implanting the device 1 in a wrapped configuration during a valve sparing procedure will now be described.

First, the aorta is opened (*i.e.*, transected) about one centimeter above the sinotubular junction. The aortic root is dissected externally to the level of the aortic annulus. The sinuses of Valsalva are resected and the coronary arteries buttons are prepared to be reimplantation in a later step.

Moreover, proximal anchoring stitch (preferably, at least six proximal anchoring stitch) are passed circumferentially through the aortic annulus from inside-out.

The sizing of the device may be performed using dedicated sizer (as previously described) or based on the leaflet dimensions.

The proximal anchoring stitches are placed at the corresponding level on the basal strip 10 (*i.e.*, they are placed at locations corresponding to the locations of the proximal anchoring stitches on the aortic annulus).

Then, the commissures tips are reimplanted with a stitch on the corresponding elongated member 20. More precisely, the commissures tips are reimplanted at the highest achievable level of the elongated member 20, *i.e.*, proximate to the free end 21.

A running suture is made to attach the remaining root tissue (along the valve insertion) to the Valsalva portion (*i.e.*, at the lobes) of the tubular portion 40 of the prosthesis 2 (*e.g.*, the Dacron graft).

The ascending aorta is resected according to the level of the aneurism and the distal anastomosis between the prosthesis 2 and the native aorta is performed.

The method for implanting the device 1 in an open configuration during an isolated repair of a tricuspid or bicuspid aortic valve will now be described.

In this case, the aorta is also transected about one centimeter above the sinotubular junction and the aortic root is dissected externally to the level of the aortic annulus.

A tunnel is created below left and coronary arteries.

The proximal anchoring stitches (for instance, felted stitches) are passed circumferentially through the aortic annulus from inside-out. The number of stitches may range between three and twelve.

The sizing of the device is performed using the sizer or based on the leaflet dimensions. Then, the device 1 of appropriate size, in its open configuration, is passed under the coronary arteries and wrapped behind the left/right commissure.

The proximal anchoring stitches are placed at the corresponding level on the basal strip 11.

Then, the commissures tips are secured on the corresponding elongated members 20, at the highest level of the elongated members 20 that they can achieve, *i.e.*, as close as possible to the free end 21.

The securing member 30 is passed circumferentially around the sinotubular junction and calibrated.

The securing member 30 is sewed to the free end 21 of the elongated members 20. Then, the aortotomy is closed.

## Claims

1. A device (1) for heart valve repair configured to be implanted around a heart valve of a subject, the heart valve comprising an annulus and at least two leaflets, the device (1) comprising:
- a base (10) configured to fit around the annulus of the heart valve, so as to limit a radially outward displacement of said annulus;
- at least two elongated members (20) extending from the base (10), each elongated member (20) extending parallel to a longitudinal axis (y) and ending in a free end (21) opposite to the base (10) and being sized to extend between the annulus of the heart valve and a commissure between the at least two leaflets;
- a securing member (30) configured to join at least two free ends (21), so as to prevent an outward displacement of said free ends (21) when the device (1) is implanted around the heart valve.

2. The device according to claim **1**, wherein the securing member (30) is a flexible band.

3. The device according to claim **1** or claim **2**, wherein each elongated member (20) is surrounded by a fabric, preferably at least one strip of fabric (22).

4. The device (1) according to any one of claims **1** to **3**, wherein the device (1) does not have an axis of symmetry.

5. The device (1) according to any one of claims **4**, wherein the base (10) is annular and the elongated members (20) are spaced apart such that the angles defined by the rays emanating from the center (O) of the annular base (10) to two consecutive elongated members (20) and are comprised between 110° and 190°.

6. The device (1) according to any one of claims **1** to **3**, wherein the device (1) is axisymmetric with respect to the longitudinal axis (y).

7. The device (1) according to any one of claims **1** to **6**, wherein the longitudinal axis (y) is perpendicular to the base (10).

8. The device (1) according to any one of claims **1** to **7**, wherein the total length of the device (1) measured along the longitudinal axis (y) is comprised between 20% and 40% of the total length of the base (10).

9. The device (1) according to any one of claims **1** to **8**, the device (1) comprising at least two adjacent arc-shaped members each comprising a curved portion (101) and two linear portions (201) terminating in two respective free ends (221), wherein at least one linear portion (201) of each arc-shaped member is joined to the linear portion (201) of the adjacent arc-shaped member at the free end (221), such that:
- the base (10) of the device (1) is formed by the curved portions (101) of the arc-shaped members, and
- the elongated members (20) of the device (1) are formed by the linear portions (201) of the arc-shaped members.

10. The device (1) according to claim **9**, wherein the two linear portions (201) of adjacent arc-shaped members are joined together along a length (L) of 75% or less of the total length (Lm) of the linear portion (201).

11. The device (1) according to claim **9** or claim **10**, wherein the device (1) is configured to be wrapped from an open configuration in which the device (1) is roughly planar and the base (10) has an elongated shape, to a wrapped configuration in which the device (1) is cylindrical and the base (10) has an annular shape.

12. The device (1) according to any one of claims **9** to **11**, wherein each pair (p) of adjacent free ends (221) except one is made by two free ends (221a) joined with continuity of material, and one pair (q) of adjacent free ends (221) is made by two free ends (221b) joined with a detachable link, such that the device (1) presents:
a. an open configuration in which the device (1) is roughly planar, and
b. a wrapped configuration in which the two adjacent free ends (221b) joined with the detachable link are linked so as to form a cylindrical device (1).

13. An aortic root replacement prosthesis (2) comprising a tubular portion (40) having a first end (41) configured to be implanted as replacement of a portion of an ascending aorta which begins at the aortic root and a second end (42) configured to be implanted as replacement of the aortic root, said second end (42) forming at least two lobes, each lobe closely fitting the space between two adjacent elongated members (20) of the device (1) according to any one of claims **1** to **12**.

14. The device (1) according to any one of claims **1** to **12**, wherein the elongated members (20) and the base (10) are made of metal or metal alloy or polymers, preferably obtained from molding or additive manufacturing from a powder or from wires.

15. The device according to claim **14**, wherein the elongated members (20) are covered with a polyester fabric.
